**Europäisches Patentamt**

**European Patent Office**

**Office européen des brevets**

(11) Publication number: **0 139 489**
**A2**

(12) # EUROPEAN PATENT APPLICATION

(21) Application number: **84306513.7**

(22) Date of filing: **25.09.84**

(51) Int. Cl.⁴: **C 12 Q 1/68**

(30) Priority: **26.09.83 US 536138**

(43) Date of publication of application:
**02.05.85 Bulletin 85/18**

(84) Designated Contracting States:
**CH DE FR GB IT LI NL SE**

(71) Applicant: **ORTHO DIAGNOSTIC SYSTEMS INC.**
**One Johnson & Johnson Plaza**
**New Brunswick New Jersey 08933(US)**

(72) Inventor: **Hansen, W. Peter**
**49 Cedar Street**
**Middlesboro Massachusetts 02346(US)**

(74) Representative: **Jones, Alan John et al,**
**CARPMAELS & RANSFORD 43 Bloomsbury Square**
**London, WC1A 2RA(GB)**

(54) Sandwich hybridization method for nucleic acid detection.

(57) Methods employing biotinylated nucleic acid probes for detecting genes or base sequences of interest in nucleic acids. The preferred embodiment employs enzyme labeled nucleic acid probe for hybridization with the nucleic acid, a biotinylated nucleic acid probe for specific hybridization with the gene or base sequence of interest and an avidin coated microparticle for immobilization thereof. Following separation, label is detected in the bound species for determining the presence of nucleic acid having the desired gene or base sequence.

# SANDWICH HYBRIDIZATION METHOD FOR NUCLEIC ACID DETECTION

This invention relates generally to the field of nucleic acid technology and specifically, relates to methods for detecting specific genes or base sequences on single stranded nucleic acid.

The recent development of genetic engineering and in particular, the manipulation of nucleic acids and genes in order to force production of desired metabolites, enzymes and the realization certain disease states are governed by identifiable base sequences, has created a demand for methods for detecting the presence of desired (or undesired) genes or base sequences in nucleic acids.

It is an object of the present invention to provide sensitive methods for the detection of such genes or base sequences.

Recently, Ranki et al. described in Gene, 21:77-85 (1983) a DNA probe sandwich assay useful for detecting a specified portion of a DNA strand. To do so, one probe is immobilized on nitrocellulose and is used to capture the sample DNA (deoxyribose nucleic acid) by hybridizing with the specific gene under investigation. Ranki also provides a second probe which is labeled and also nonspecific to the gene of interest.

Ranki's method, however, suffers from several disadvantages including variations in specific and nonspecific binding characteristic of extended solid support systems such as those employing nitrocellulose. The repeatability of such solid support preparations has

CRL-3

been shown to be a key difficulty in the production of coated macroscopic surfaces such as coated test tubes in the field of immunoassays. These variations ultimately limit the sensitivity of the assay to the range of the variations. Accordingly, it may be expected that similar problems will be associated with Ranki's method.

It is an object of the present invention to avoid these limitations by providing a method capable of more repeatable commercial production.

## Summary of the Invention

In accordance with the objectives of the present invention, methods are provided for detecting nucleic acids having a desired gene or base sequence comprising the steps of providing the nucleic acid to be tested in a single stranded form and thereafter contacting it with a labeled nucleic acid probe specific for a given section of the nucleic acid strand. A biotinylated nucleic acid probe specific for a different portion of the nucleic acid strand, is bonded to an avidin coated microparticle. The strand having the labeled probe hybridized thereto is then mixed with the thusly prepared microparticles. Binding of the DNA strand to the microparticle occurs through the biotinylated probe that coats the microparticle. The avidin-biotin coupling is sufficiently strong to permit the separation of microparticle bound DNA from unbound material. The bound material is subsequently assayed for label. The presence of the label is indicative of the presence of a strand having both portions for which the biotinylated and labeled probes are specific. Either one of these

CRL-3                                   2

sections may be the gene or base sequence of interest. Alternately, the decrease of label in solution may be monitored and related to the presence of the gene or base sequence of interest.

## Brief Summary of the Drawing

In accordance with the principles of the present invention, further understanding may be had by reference to the figure wherein:

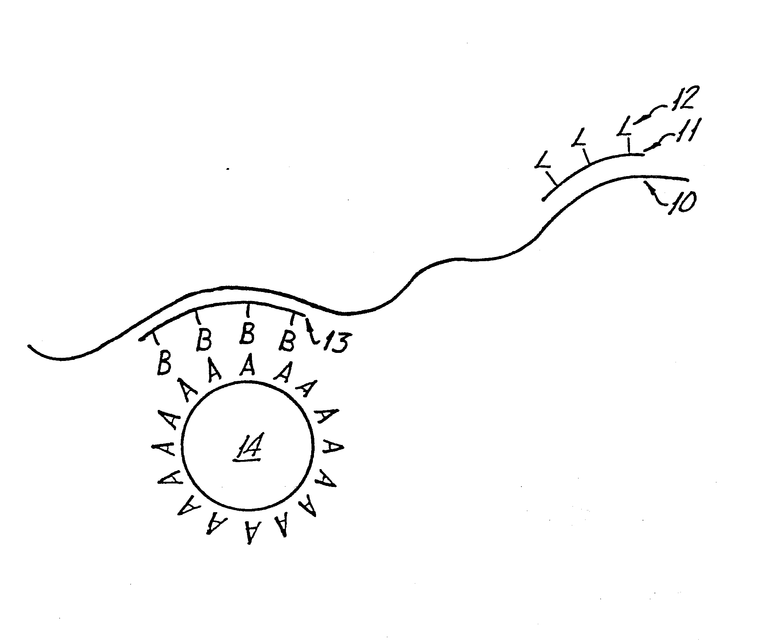

FIG. 1 artistically shows the labeling and immobilization of a single stranded nucleic acid.

## Detailed Description of the Drawing and Best Mode

With reference to FIG. 1, single stranded nucleic acid 10 is provided and may be in the form of ribose nucleic acid or deoxyribose nucleic acid which has at least been reduced to a single stranded form by any of a number of well known techniques such as heating or adding a strong base. The nucleic acid strand 10 is reacted with both a nucleic acid probe 11 having a label 12, and a biotinylated nucleic acid probe 13. These reactions will be carried out in solution and preferably with an excess of labeled nucleic acid probe 11.

Also provided are microparticles 14 coated with avidin. The microparticles may be made from a variety of materials including glass, nylon, polymethacrylate, other polymeric material, or biological cells. Such microparticles may be readily obtained from a variety of sources including, for instance, Polysciences Inc.,

Pennsylvania. An avidin coating may be readily prepared by physical adsorption or direct chemical means such as coupling via the N-hydroxysuccinimide active ester (Manning et al., Biochemistry 16:1364-1370, 1977) in the case of glass, or a carbodiimide coupling in the case of nylon (Jasiewicz et al., Exp. Cell Res. 100:213-217, 1976).

If it is desired to avoid the use of avidin-biotin coupling mechanisms with microparticles, one may instead employ nitrocellulose microparticles, as opposed to Ranki's nitrocellulose sheets, to obtain direct chemical bonding of single stranded nucleic acid to the solid phase. Although the mechanism of attachment is as yet unknown, it has been learned that after attachment of the nucleic acid, remaining attachment sites must be blocked by reaction with additional DNA such as salmon testes DNA prior to utilization of the thusly prepared solid phase surface.

The mixture of nucleic acid having biotinylated probe hybridized therewith with the avidin coated microparticles or beads will, due to the strong avidin-biotin attraction, result in the immobilization of the nucleic acid hybrid pair. The nucleic acid may then be separated from the unbound materials by centrifugation, filtration, or washing steps such as those that may be employed with heterogeneous immunoassay techniques.

The label will preferably be selected in order to be optically detectable with readily available instruments for yielding acceptable sensitivities. Such labels will include, for instance, fluorophores, i.e. those that fluoresce under suitable excitation wavelengths, chemiluminescent labels which luminesce under appropriate chemical conditions, or any of the enzyme associated labeling mechanisms whereby a detectable product is produced by the action of an enzyme label upon a substrate. Such a product may, for example, be selected so as to be readily detected colorimetrically. Although less preferred, the label may instead be a radioisotope. Typically, however, isotopic labels are less preferable as they require special handling due to significant health risks associated with such labels, exhibit finite shelf-life, and require expensive equipment such as scintillation or gamma counters. Still other types of labels are contemplated and would include for instance, materials possessing detectable electromagnetic properties.

In addition to the microparticles described above, it is also contemplated that the methods provided herein may be employed with other types of solid phase surfaces including, for instance, the walls of a microtiter tray, paddles, or other macroscopic surfaces such as disks or tapes. Such disks or tapes may have the avidin coatings arranged thereon in a pattern. Such patterns are useful, for example, in the class of instruments employing synchronous detection.

Various well known techniques are available for coupling labels with nucleic acid probes. One such technique is described by Langer et al. in Proc. Natl. Acad. Sci. 78:6633-6637, 1981.

A preferred order of reaction employing avidin and biotin to couple label 12 to probe 11 would involve the following steps. First, biotinylated probe 11 is reacted with nucleic acid strand 10 followed by reaction of avidin coupled label 12 with biotinylated probe 11 to form a first mixture. Separately, avidin coated microparticles 14 are reacted with biotinylated probe 13 to form a second mixture. The first and second mixtures are reacted to permit formation of complexes as depicted in FIG. 1. These immobilized complexes are then separated and either the label associated therewith or the free label remaining in the solution measured. An example of a nonpreferred order of reaction would permit binding of a biotinylated probe 11 with an avidin coated microparticle 14.

The order of reaction between the single stranded nucleic acid, the biotinylated nucleic acid probe (not employing avidin-biotin), the labeled nucleic acid probe, and the avidin coated solid phase support may be varied in order to suit the needs of the investigator. For instance, it may be found desirable to react the biotinylated nucleic acid probe with the avidin coated solid phase surface prior to adding the nucleic acid strand. The nucleic acid strand may be either previously reacted with the labeled nucleic acid probe or subsequently reacted therewith. A nonpreferred order of reaction would permit binding of a biotinylated probe 11 with an avidin coated microparticle 14.

In another embodiment, it is contemplated that one may wish to provide microparticles coated with biotinylated nucleic acid probe segments useful for any assay. These microparticles could then be customized for any given assay by reacting a longer nucleic acid probe, or linking probe, with the biotinylated probe so that the section of the linking probe that is homologous to the biotinylated probe forms a double stranded nucleic acid section. The remaining portion of the linking probe would preferably be homologous to the gene being sought in the nucleic acid under analysis. Preparation of the solid phase portion in this manner, particularly with respect to microparticles, would allow manufacturing to occur on a large scale and would supply essential components useful for any nucleic acid assay.

Employment of microparticles is greatly preferred since it is to be expected that there may be significant variation of immobilized probe 13 present on microparticles 14, however, if a large quantity of microparticles 14 are prepared and randomly divided among a number of assay tests, then the variation in total probe 13 from test to test will be reduced in proportion to the reciprocal of the square root of the number of particles used per test.

The skilled investigator will readily appreciate that separation of unbound nucleic acid strands in those embodiments employing solid phase surfaces such as microtiter well walls, or macroscopic surfaces and the like, will be readily accomplished by gentle washing steps.

Nucleic acid probes such as those contemplated in the present invention, may be readily obtained from a variety of sources including Enzo-Biochem, New York, NY.

RL-3

7

It will be readily understood by those skilled in the art that numerous alterations of the above may be had without departing from either the spirit or the scope of the present invention.

L-3

8

CLAIMS

1.    A method for detecting the presence of nucleic acid containing a gene or base sequence of interest comprising:

    a)    providing the nucleic acid to be tested in a single strand form;

    b)    contacting a biotinylated nucleic acid probe specific for a first gene or base sequence of said nucleic acid with an avidin coated solid phase support;

    c)    reacting said nucleic acid with an excess of labeled nucleic acid probe specific for a second gene or base sequence of said nucleic acid and permitting any hybridization of said labeled probe and said nucleic acid to occur;

    d)    reacting said nucleic acid with said biotinylated nucleic acid probe and permitting any hybridization of said biotinylated probe and said nucleic acid to occur;

    e)    separating said unbound nucleic acid from said bound nucleic acid; and

    f)    detecting the label associated with said bound nucleic acid or the decrease in the amount of label in solution for determining the presence of nucleic acid containing the gene or base sequence of interest.

9

-10-

2. The method as provided in Claim 1 wherein the order of reaction is as provided in Claim 1.

3. A method for detecting the presence of nucleic acid containing a gene or base sequence of interest comprising:

   a) providing the nucleic acid to be tested in a single stranded form;

   b) further providing an avidin coated solid phase support which has been reacted with a first biotinylated nucleic acid probe to form a first solid phase species;

   c) contacting said first solid phase species with a nucleic acid probe linking means having a first portion which is reactive with at least some part of said first biotinylated nucleic acid probe and a second portion reactive with a first gene or base sequence of said nucleic acid and permitting any hybridization to occur to form a first mixture;

   d) reacting said nucleic acid with an excess of labeled nucleic acid probe specific for a second gene or base sequence of said nucleic acid and permitting any hybridization of said labeled probe and said nucleic acid to occur to form a second mixture;

   e) reacting said first mixture with said second mixture;

   f) separating solid phase immobilized nucleic acid from unbound nucleic acid; and

   g) detecting the label associated with said immobilized nucleic acid portion for determining the presence of nucleic acid containing the gene or base sequence of interest.

4. The method as provided in Claim 3 wherein the order of reaction is as set forth in Claim 3.

5. The method of any one of Claims 1 to 4 wherein the solid phase is selected from the group consisting of microparticles and macroscopic surfaces.

6. The method as provided in Claim 5 wherein the solid phase comprises microparticles.

7. The method as provided in Claim 5 wherein the solid phase comprises macroscopic surfaces.

8. The method as provided in Claim 7 wherein said biotinylated label is immobilized on said macroscopic surface in a specific spatial pattern.

9. The method of any one of Claims 1 to 8 wherein the detecting step comprises detecting fluorescence.

10. The method of any one of Claims 1 to 8 wherein the detecting step comprises detecting chemiluminescence.

11. The method of any one of Claims 1 to 8 wherein the detecting step comprises detecting an isotopic label.

12. The method of any one of Claims 1 to 8 wherein the detecting step comprises detecting products of enzymatic labels.

13. The method of any one of Claims 1 to 8 wherein the detecting step comprises detecting light scatter.

14. The method of any one of Claims 1 to 4 wherein the detecting step comprises detecting by colorimetry.

15. The method of any one of Claims 1 to 14 wherein the providing step further comprises splitting double stranded nucleic acid into single stranded nucleic acid if said nucleic acid is deoxyribose nucleic acid.

-12-

16. A method for detecting the presence of nucleic acid containing a gene or base sequence of interest comprising:

a) providing the nucleic acid to be tested in a single strand form;

b) attaching a nucleic acid probe specific for a gene or base sequence of said nucleic acid to a nitrocellulose microparticle;

c) reacting said nucleic acid with an excess of labeled nucleic acid probe specific for a first gene or base sequence of said nucleic acid and permitting any hybridization of said labeled probe and said nucleic acid to occur;

d) reacting said nucleic acid with said nitrocellulose attached nucleic acid probe and permitting any hybridization to occur;

e) separating said unbound nucleic acid from said bound nucleic acid.

f) detecting the label associated with said bound nucleic acid or the decrease in the amount of label in solution for determining the presence of nucleic acid containing the gene or base sequence of interest.

0139489